# EUROPÄISCHE PATENTANMELDUNG

(11) **EP 0 672 387 A1**
(43) Veröffentlichungstag der Anmeldung: **20.09.1995**
(21) Anmeldenummer: 95100837.4
(22) Anmeldetag: 23.01.1995
(51) Int. Cl.: A61B 17/36, A61C 1/00

(54) **Medizinisches Handstück**

(30) Priorität: 25.01.1994 DE 4401989
(71) Anmelder: AESCULAP AG, D-78532 Tuttlingen (DE)
(72) Erfinder: Vogler, Klaus, D 90542 Eckental (DE); Reindl, Maximilian, D 90562 Heroldsberg (DE); Donitzky, Christof, D 90542 Eschenau (DE)
(74) Vertreter: Hoeger, Stellrecht & Partner

(57) **Zusammenfassung**

Um ein medizinisches Handstück, insbesondere für die Dentalmedizin, zur Ausrichtung einer von einer Laserstrahlungsquelle emittierten Laserstrahlung auf einen zu behandelnden Körperteil mit einem Gehäuse, das eine Austrittsöffnung für die Laserstrahlung aufweist, so auszugestalten, daß Verluste beim Übertragen der Laserstrahlung von einer Laserstrahlungsquelle zum medizinischen Handstück und technisch aufwendige Ein- und Auskopplungen der Laserstrahlung vermieden werden sowie die Handhabbarkeit des medizinischen Handstücks erhöht wird, wird vorgeschlagen, daß die Laserstrahlungsquelle im Gehäuse angeordnet ist.

## Beschreibung

Die Erfindung betrifft ein medizinisches Handstück, insbesondere für die Dentalmedizin, zur Ausrichtung einer von einer Laserstrahlungsquelle emittierten Laserstrahlung auf einen zu behandelnden Körperteil, mit einem Gehäuse, das eine Austrittsöffnung für die Laserstrahlung aufweist.

Derartige medizinische Handstücke können beispielsweise dazu verwendet werden, die von einem Er:YAG-Laser emittierte Laserstrahlung auf einen Zahn zu richten, um im Rahmen einer Kariesbehandlung Zahnsubstanz abzutragen. Laserstrahlung wird in zunehmendem Maße allgemein dazu verwendet, Körpergewebe zu koagulieren oder zu verdampfen.

Die Laserstrahlung wird dabei von einer Laserstrahlungsquelle emittiert und anschließend in eine Strahlführungseinheit eingekoppelt. Die Strahlführungseinheit kann dabei als ein mehrere Gelenke aufweisender Strahlarm oder als Lichtleitfaser ausgebildet sein. Mit Hilfe der Strahlführungseinheit wird die Laserstrahlung in das medizinische Handstück geführt, so daß sie von einer Behandlungsperson auf den zu behandelnden Körperteil gerichtet werden kann.

Die Ausbildung der Strahlführungseinheit als Strahlarm begrenzt ebenso wie die Ausbildung als Lichtleitfaser die Handhabbarkeit des medizinischen Handstücks. Mit dem Strahlarm sind ein bestimmtes Gewicht, eine Steifigkeit und mechanische Beweglichkeitsschranken verbunden, die die Handhabung des medizinischen Handstücks ebenso beschränken wie Lichtleitfasern, die insbesondere beim Einsatz von Laserstrahlung im mittleren Infrarotbereich, das heißt im Wellenlängenbereich von etwa 2 bis 3 µm, aus einem Material mit relativ großem minimalen Biegeradius und hoher Bruchgefahr gebildet sind. Bei der Übertragung der Laserstrahlung mit Hilfe einer Strahlführungseinheit ist eine im allgemeinen technisch aufwendige Ein- und Auskopplung der erzeugten Laserstrahlung erforderlich, und im Falle der Verwendung von Lichtleitfasern kann nur eine begrenzte Strahlungsenergie übertragen werden, da sonst die Gefahr der Zerstörung der Lichtleitfasern besteht. Beide Ausführungsformen der Strahlführungseinheit haben außerdem den Nachteil, daß es bei der Übertragung der Laserstrahlung von der Laserstrahlungsquelle zum medizinischen Handstück zu Strahlungsverlusten kommt.

Aufgabe der Erfindung ist es, ein gattungsgemäßes medizinisches Handstück so auszugestalten, daß die Handhabbarkeit verbessert und Strahlungsverluste sowie technisch aufwendige Ein- und Auskopplungen der Laserstrahlung vermieden werden.

Diese Aufgabe wird bei einem medizinischen Handstück der eingangs beschriebenen Art erfindungsgemäß dadurch gelöst, daß die Laserstrahlungsquelle im Gehäuse angeordnet ist.

Beim erfindungsgemäßen medizinischen Handstück ist die Laserstrahlungsquelle in das Gehäuse integriert, so daß eine Strahlführungseinheit für die auf den zu behandelnden Körperteil zu richtende Laserstrahlung vollständig entfällt. Die Handhabung des medizinischen Handstücks ist deshalb nicht durch mechanische Beweglichkeitsschranken eines Strahlarms oder begrenzte Biegeradien einer Lichtleitfaser zur Übertragung von Infrarotstrahlung begrenzt.

Vorteilhaft ist es, das Gehäuse mit einem Griffstück und einem die Austrittsöffnung umfassenden, mit dem Griffstück lösbar verbindbaren Endstück auszugestalten. Auf diese Weise ist es möglich, verschiedene alternativ montierbare Endstücke zu verwenden, die sterilisierbar ausgebildet und unterschiedlichen medizinischen Erfordernissen gerecht werden können. Die Endstücke können beispielsweise eine derartige Form aufweisen, daß auch schwer zugängliche Stellen innerhalb des Mundraums eines Patienten mit Laserstrahlung beaufschlagt werden können. Eine derartige Ausgestaltung bewirkt somit eine weitere Verbesserung der Handhabbarkeit.

Es ist außerdem vorteilhaft, wenn das medizinische Handstück optische Mittel zur Strahlführung der Laserstrahlung von der Laserstrahlungsquelle zur Austrittsöffnung umfaßt. Es können beispielsweise Umlenkspiegel zum Einsatz kommen, mit deren Hilfe die Laserstrahlung auf schwer zugängliche Körperpartien gerichtet werden kann.

Bei einer vorteilhaften Ausführungsform der Erfindung ist vorgesehen, daß das medizinische Handstück ein die Laserstrahlung auf einen außerhalb des Gehäuses positionierten Brennpunkt bündelndes Fokussierelement umfaßt. Dadurch läßt sich die Laserstrahlung auf ein im wesentlichen punktförmiges Gebiet konzentrieren und beispielsweise ein eng begrenzter Bereich eines Zahns behandeln.

Bei einer vorteilhaften Ausführungsform ist vorgesehen, daß das medizinische Handstück ein Frequenzwandlungselement umfaßt, so daß die Frequenz der erzeugten Laserstrahlung beispielsweise verdoppelt und somit den speziellen Absorptionseigenschaften eines zu behandelnden Körperteils angepaßt werden kann.

Die im medizinischen Handstück integrierte Laserstrahlungsquelle umfaßt in einer vorteilhaften Ausführungsform der Erfindung ein mit Hilfe von einer Pumpstrahlungsquelle emittierter, optischer Pumpstrahlung anregbares laseraktives Material. Das laseraktive Material kann in bekannter Weise zwischen einem teildurchlässigen Auskoppelspiegel und einem hochreflektierenden Endspiegel positioniert sein.

Bei einer vorteilhaften Ausführungsform ist vorgesehen, daß das laseraktive Material ein beispielsweise stabförmiger Festkörperlaserkristall ist.

Eine Miniaturisierung des medizinischen Handstücks läßt sich dadurch erzielen, daß eine erste Stirnseite des Festkörperlaserkristalls eine die Laserstrahlung reflektierende Verspiegelung aufweist, so daß dadurch ein zusätzlicher Endspiegel entfallen kann, da die von einem Bereich innerhalb des Festkörperlaserkristalls ausgehende und auf die erste Stirnseite auftreffende Laserstrahlung aufgrund der reflektierenden Verspiegelung in den Innenbereich zurückgerichtet wird.

Eine weitere Miniaturisierung ergibt sich dadurch, daß eine zweite Stirnseite des Festkörperlaserkristalls eine die Laserstrahlung teilweise durchlassende Verspiegelung aufweist, so daß auch der Auskoppelspiegel entfallen und somit die Ausdehnung der Laserstrahlungsquelle beträchtlich reduziert werden kann.

Bei einer weiteren vorteilhaften Ausführungsform ist vorgesehen, daß die zweite Stirnseite des Festkörperlaserkristalls die aus dem Kristall austretende Laserstrahlung fokussierend ausgebildet ist. Die zweite Stirnseite des Festkörperlaserkristalls weist in diesem Fall eine derartige Formgebung auf, daß die beim Austritt der Laserstrahlung aus dem Kristall auftretende Brechung eine Fokussierung der emittierten Laserstrahlung zur Folge hat. Auf diese Weise lassen sich zusätzliche optische Mittel zur Fokussierung der Laserstrahlung vermeiden, so daß die mechanische Ausdehnung des medizinischen Handstücks reduziert werden kann.

Der Festkörperlaserkristall kann beispielsweise als YAG-Kristall ausgebildet sein oder auch als GGG(Gd₃Ga₅0₁₂)-, GSGG(Gd₃Sc₂Ga₃O₁₂)- oder YSGG(Y₃Sc₂Ga₃O₁₂)-Kristall.

Bei einer bevorzugten Ausführungsform ist vorgesehen, daß das laseraktive Material als Faserlaser ausgebildet ist. Die Ausbildung des laseraktiven Materials in Form einer biegsamen Faser hat den Vorteil, daß es aufgewickelt und somit sehr lang ausgebildet sein kann, ohne daß sich die für die Verstärkung der Laserstrahlung maßgebliche Länge des laseraktiven Materials störend auf die Größe des medizinischen Handstücks auswirkt.

Als Wirtsmaterial für den Faserlaser kann beispielsweise ZBLAN oder Glas verwendet werden.

Bei einer bevorzugten Ausführungsform der Erfindung ist das laseraktive Material mit Erbium dotiert. Durch die Dotierung mit Erbium läßt sich Laserstrahlung mit einer Wellenlänge von 2,94 µm erzielen, die insbesondere für die Abtragung von Zahnsubstanz besonders effektiv ist.

Bei einer weiteren vorteilhaften Ausführungsform ist das Lasermaterial mit Holmium dotiert, so daß insbesondere Laserstrahlung mit einer Wellenlänge von 2,06 µm erzeugt werden kann.

Weitere vorteilhafte Ausführungsformen der Erfindung ergeben sich durch Dotierung des laseraktiven Materials mit Thulium oder Neodym.

Eine zusätzliche Miniaturisierung des medizinischen Handstücks ergibt sich dadurch, daß die für die Anregung des laseraktiven Materials erforderliche optische Pumpstrahlung über eine in das Gehäuse einmündende Lichtleitfaser dem laseraktiven Material zugeführt wird. Da für die Pumpstrahlung im allgemeinen keine Infrarotstrahlung erforderlich ist, läßt sich für die Lichtleitfaser eine gewöhnliche Quarzfaser verwenden, die eine größere mechanische Stabilität aufweist als für die Übertragung von Infrarotstrahlung geeignete Lichtleitfasern, so daß die Handhabung des medizinischen Handstücks durch die Lichtleitfaser für die Pumpstrahlung nicht wesentlich beeinflußt wird.

Besonders vorteilhaft ist es, neben der Laserstrahlungsquelle auch die Pumpstrahlungsquelle im Gehäuse anzuordnen, da dadurch die Verwendung einer Lichtleitfaser vollständig entfällt.

Als Pumpstrahlungsquelle kann sowohl eine Blitzlampe verwendet werden als auch ein Diodenlaser. Der Diodenlaser hat zusätzlich den Vorteil, daß damit sowohl ein transversales als auch ein longitudinales Pumpen ermöglicht wird. Bei einer longitudinalen Pumpanordnung läßt sich das medizinische Handstück besonders dünn ausgestalten.

Indem bei einer weiteren vorteilhaften Ausführungsform der Erfindung als Laserstrahlungsquelle ein Diodenlaser verwendet wird, entfällt die Verwendung einer Pumpstrahlungsquelle vollständig, so daß das medizinische Handstück ein besonders geringes Gewicht und kleine Ausmaße aufweisen kann.

Die Handhabbarkeit des medizinischen Handstücks läßt sich außerdem dadurch steigern, daß dessen Energieversorgung durch eine im Gehäuse angeordnete Batterie erfolgt, da dadurch keine elektrischen Zuführungskabel erforderlich sind.

Bei einer besonders bevorzugten Ausführungsform der Erfindung ist vorgesehen, daß das medizinische Handstück einen sichtbaren Zielstrahl in den Strahlengang der Laserstrahlung einkoppelnde optische Mittel umfaßt. Da Körpergewebe aufgrund des Wassergehalts im infraroten Spektralbereich zwischen 2 und 3 µm eine besonders hohe Absorption aufweist, wird in vielen Fällen für die Behandlung eines Körperteils infrarote Laserstrahlung verwendet. Um der Bedienungsperson trotz der für das menschliche Auge unsichtbaren Infrarotstrahlung die Möglichkeit zu geben, die Richtung der aus der Austrittsöffnung des medizinischen Handstücks austretenden infraroten Laserstrahlung zu ermitteln, wird in den Strahlengang der Laserstrahlung ein sichtbarer Zielstrahl eingekoppelt, dessen Ausbreitungsrichtung mit der Richtung der Laserstrahlung übereinstimmt und von der Bedienungsperson festgestellt werden kann.

Dabei kann vorgesehen sein, daß der Zielstrahl über eine in das Gehäuse einmündende Lichtleitfaser dem medizinischen Handstück zugeführt wird.

Bei einer besonders vorteilhaften Ausführungsform der Erfindung ist vorgesehen, daß im Gehäuse eine Strahlungsquelle für den Zielstrahl angeordnet ist, so daß die Handhabbarkeit des medizinischen Handstücks auch bei Verwendung eines Zielstrahls nicht durch eine Lichtleitfaser beeinflußt wird. Als Strahlungsquelle für den Zielstrahl kann beispielsweise ein Diodenlaser verwendet werden.

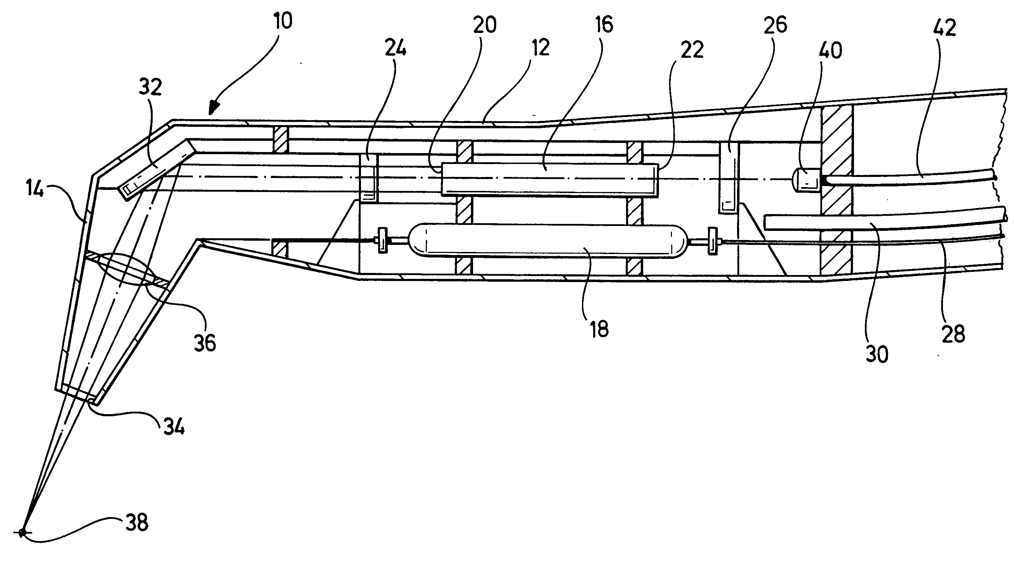

Die nachfolgende Beschreibung einer bevorzugten Ausführungsform der Erfindung dient im Zusammenhang mit der Zeichnung der näheren Erläuterung. Die Zeichnung zeigt einen Querschnitt eines Dentalhandstücks, in das eine Laserstrahlungsquelle integriert ist.

Das Dentalhandstück wird begrenzt von einem im wesentlichen L-förmigen Gehäuse 10 mit einem langen Schenkel 12 und einem kurzen Schenkel 14. Im langen Schenkel 12 des Gehäuses 10 ist parallel zur Längsachse des langen Schenkels 12 als laseraktives Material ein stabförmiger Festkörperlaserkristall 16 in Form eines mit Erbium dotierten YAG-Kristalls angeordnet, sowie parallel zum Festkörperlaserkristall 16 als Pumpstrahlungsquelle eine optische Blitzlampe 18. Parallel zu den Stirnflächen 20, 22 des Festkörperlaserkristalls 16 sind im Abstand zu diesen ein teildurchlässiger Auskoppelspiegel 24 beziehungsweise ein hochreflektierender Endspiegel 26 im langen Schenkel 12 des Gehäuses 10 positioniert.

Die Blitzlampe 18 ist über eine elektrische Versorgungsleitung 28 mit einer in der Zeichnung nicht dargestellten Versorgungseinheit für das Dentalhandstück verbunden und wird von dieser mit elektrischer Energie versorgt. Zusätzlich ist das Dentalhandstück über eine Kühlwasserleitung 30 mit der Versorgungseinheit verbunden, wobei die Kühlwasserleitung 30 und die Versorgungsleitung 28 über das in der Zeichnung nicht dargestellte, dem kurzen Schenkel 14 abgewandte Ende des langen Schenkels 12 in das Gehäuse 10 einmünden.

Die parallel zueinander ausgerichteten Auskoppel- und Endspiegel 24, 26 definieren eine Resonatoranordnung, in der der Festkörperlaserkristall 16 positioniert ist, der durch von der Blitzlampe 18 ausgesandte optische Pumpstrahlung zur Emission von Laserstrahlung angeregt wird. Während der Endspiegel 26 die Laserstrahlung vollständig reflektiert, ist der Auskoppelspiegel 24 teilweise durchlässig für die Laserstrahlung, so daß diese aus der Resonatoranordnung austreten kann, um anschließend auf einen ungefähr unter 45° zur Laserstrahlrichtung positionierten Umlenkspiegel 32 zu treffen und von diesem durch das als Austrittsöffnung 34 ausgebildete offene Ende des kurzen Schenkels 14 aus dem Gehäuse 10 herausgeführt zu werden. Im Bereich zwischen dem Umlenkspiegel 32 und der Austrittsöffnung 34 ist im Strahlengang der Laserstrahlung als Fokussierelement eine Konvexlinse 36 angeordnet, durch die die Laserstrahlung auf einen außerhalb des Gehäuses 10 positionierten Brennpunkt 38 fokussiert wird.

Im Abstand von der dem Festkörperlaserkristall 16 abgewandten Seite des Endspiegels 26 ist im langen Schenkel 12 des Gehäuses 10 in Höhe der Mittelachse des Festkörperlaserkristalls 16, die mit der Mittelachse der aus den beiden Spiegeln 24 und 26 gebildeten Resonatoranordnung übereinstimmt, als Strahlungsquelle für einen Zielstrahl ein Diodenlaser 40 angeordnet, der über eine Diodenversorgungsleitung 42 mit der in der Zeichnung nicht dargestellten Versorgungseinheit des Dentalhandstücks verbunden ist. Der Diodenlaser 40 emittiert koaxial zur Mittelachse des Festkörperlaserkristalls 16 - und damit auch koaxial zur Strahlachse der vom Festkörperlaserkristall 16 ausgehenden Laserstrahlung - einen sichtbaren Zielstrahl. Der Auskoppelspiegel 24 und der Endspiegel 26 sind ebenso wie der Festkörperlaserkristall 16 für den sichtbaren Zielstrahl durchlässig, so daß in Strahlrichtung der durch den Auskoppelspiegel 24 hindurchtretenden Laserstrahlung der Strahlengang des sichtbaren Zielstrahls mit der infraroten Laserstrahlung übereinstimmt. Dies ermöglicht einer Bedienungsperson, die Richtung der aus der Austrittsöffnung 34 austretenden infraroten Laserstrahlung anhand des sichtbaren Zielstrahls festzustellen.

Da die Laserstrahlungsquelle in Form des Festkörperlaserkristalls 16 ebenso wie die Pumpstrahlungsquelle in Form der Blitzlampe 18 und die Strahlungsquelle für den Zielstrahl (Diodenlaser 40) im Dentalhandstück integriert sind, entfällt die sonst übliche Strahlführungseinheit zur Übertragung der Laserstrahlung von einer separaten Laserstrahlungsquelle zum Dentalhandstück, wodurch insbesondere dessen Handhabbarkeit erleichtert wird und Übertragungsverluste vermieden werden.

## Patentansprüche

1. Medizinisches Handstück, insbesondere für die Dentalmedizin, zur Ausrichtung einer von einer Laserstrahlungsquelle emittierten Laserstrahlung auf einen zu behandelnden Körperteil mit einem Gehäuse, das eine Austrittsöffnung für die Laserstrahlung aufweist, dadurch gekennzeichnet, daß die Laserstrahlungsquelle im Gehäuse (10) angeordnet ist.

2. Medizinisches Handstück nach Anspruch 1, dadurch gekennzeichnet, daß das Gehäuse (10) ein Griffstück und ein die Austrittsöffnung (34) umfassendes, mit dem Griffstück lösbar verbindbares Endstück aufweist.

3. Medizinisches Handstück nach Anspruch 1 oder 2, dadurch gekennzeichnet, daß das medizinische Handstück optische Mittel (32) zur Strahlführung der Laserstrahlung von der Laserstrahlungsquelle zur Austrittsöffnung (34) umfaßt.

4. Medizinisches Handstück nach Anspruch 1, 2 oder 3, dadurch gekennzeichnet, daß das medizinische Handstück ein die Laserstrahlung auf einen außerhalb des Gehäuses (10) positionierten Brennpunkt (38) bündelndes Fokussierelement (36) umfaßt.

5. Medizinisches Handstück nach einem der Ansprüche 1 bis 4, dadurch gekennzeichnet, daß das medizinische Handstück ein Frequenzwandlungselement umfaßt.

6. Medizinisches Handstück nach einem der voranstehenden Ansprüche, dadurch gekennzeichnet, daß die Laserstrahlungsquelle ein mit Hilfe von einer Pumpstrahlungsquelle (18) emittierter, optischer Pumpstrahlung anregbares laseraktives Material (16) umfaßt.

7. Medizinisches Handstück nach Anspruch 6, dadurch gekennzeichnet, daß das laseraktive Material ein Festkörperlaserkristall (16) ist.

8. Medizinisches Handstück nach Anspruch 7, dadurch gekennzeichnet, daß eine erste Stirnseite (22) des Festkörperlaserkristalls (16) eine die Laserstrahlung reflektierende Verspiegelung aufweist.

9. Medizinisches Handstück nach Anspruch 7 oder 8, dadurch gekennzeichnet, daß eine zweite Stirnseite (20) des Festkörperlaserkristalls (16) eine die Laserstrahlung teilweise durchlassende Verspiegelung aufweist.

10. Medizinisches Handstück nach Anspruch 7, 8 oder 9, dadurch gekennzeichnet, daß die zweite Stirnseite (20) des Festkörperlaserkristalls (16) die aus dem Kristall austretende Laserstrahlung fokussierend ausgebildet ist.

11. Medizinisches Handstück nach einem der Ansprüche 7 bis 10, dadurch gekennzeichnet, daß der Festkörperlaserkristall (16) ein YAG-Kristall ist.

12. Medizinisches Handstück nach einem der Ansprüche 7 bis 10, dadurch gekennzeichnet, daß der Festkörperlaserkristall (16) ein GGG-, GSGG- oder YSGG-Kristall ist.

13. Medizinisches Handstück nach Anspruch 6, dadurch gekennzeichnet, daß das laseraktive Material (16) als Faserlaser ausgebildet ist.

14. Medizinisches Handstück nach Anspruch 13, dadurch gekennzeichnet, daß das Wirtsmaterial des Faserlasers aus ZBLAN oder Glas gebildet ist.

15. Medizinisches Handstück nach einem der Ansprüche 6 bis 14, dadurch gekennzeichnet, daß das laseraktive Material (16) mit Erbium dotiert ist.

16. Medizinisches Handstück nach einem der Ansprüche 6 bis 15, dadurch gekennzeichnet, daß das laseraktive Material (16) mit Holmium dotiert ist.

17. Medizinisches Handstück nach einem der Ansprüche 6 bis 16, dadurch gekennzeichnet, daß das laseraktive Material (17) mit Thulium dotiert ist.

18. Medizinisches Handstück nach einem der Ansprüche 6 bis 17, dadurch gekennzeichnet, daß das laseraktive Material (16) mit Neodym dotiert ist.

19. Medizinisches Handstück nach einem der Ansprüche 6 bis 18, dadurch gekennzeichnet, daß die Pumpstrahlung dem laseraktiven Material (16) über eine in das Gehäuse (10) einmündende Lichtleitfaser zugeführt wird.

20. Medizinisches Handstück nach einem der Ansprüche 6 bis 18, dadurch gekennzeichnet, daß die Pumpstrahlungsquelle (18) im Gehäuse (10) angeordnet ist.

21. Medizinisches Handstück nach Anspruch 19 oder 20, dadurch gekennzeichnet, daß die Pumpstrahlungsquelle eine Blitzlampe (18) umfaßt.

22. Medizinisches Handstück nach Anspruch 19 oder 20, dadurch gekennzeichnet, daß die Pumpstrahlungsquelle (18) einen Diodenlaser umfaßt.

23. Medizinisches Handstück nach einem der Ansprüche 1 bis 5, dadurch gekennzeichnet, daß die Laserstrahlungsquelle einen Diodenlaser umfaßt.

24. Medizinisches Handstück nach einem der voranstehenden Ansprüche, dadurch gekennzeichnet, daß die Energieversorgung des medizinischen Handstücks durch eine im Gehäuse angeordnete Batterie erfolgt.

25. Medizinisches Handstück nach einem der voranstehenden Ansprüche, dadurch gekennzeichnet, daß das medizinische Handstück einen sichtbaren Zielstrahl in den Strahlengang der Laserstahlung einkoppelnde optische Mittel umfaßt.

26. Medizinisches Handstück nach einem der voranstehenden Ansprüche, dadurch gekennzeichnet, daß in das Gehäuse (10) eine den sichtbaren Zielstrahl führende Lichtleitfaser einmündet.

27. Medizinisches Handstück nach einem der voranstehenden Ansprüche, dadurch gekennzeichnet, daß im Gehäuse (10) eine Strahlungsquelle (40) für den Zielstrahl angeordnet ist.
